# EUROPÄISCHE PATENTANMELDUNG

(11) **EP 1 174 162 A1**
(43) Veröffentlichungstag der Anmeldung: **23.01.2002**
(21) Anmeldenummer: 00115439.2
(22) Anmeldetag: 18.07.2000
(51) Int. Cl.: A61N 1/16

(54) **Textilgewebe mit EMF - dämpfenden Materialien und ihre Verwendung zur Abschirmung von HF-Strahlung**

(71) Anmelder: Block, Lutz Henning, Prof. Dr., 79312 Emmendingen (DE)
(72) Erfinder: Block, Lutz Henning, Prof. Dr., 79312 Emmendingen (DE)
(74) Vertreter: Benz, Jürgen, Dr.

(57) **Zusammenfassung**

Die Erfindung betrifft Textilgewebe im allgemeinen, insbesondere aber Kleidungstücke jeglicher Art, welche Materialien enthalten, die elektromagnetische Felder (EMF) zu dämpfen vermögen, und ihre Verwendung zur Abschirmung von hochfrequenter Strahlung (HF-Strahlung). Derartige Gewebe sind somit hervorragend geeignet, Strahlung, welche von Radio- oder Mikrowellen emittierenden Geräten und Einrichtungen, insbesondere Mobiltelefone, Handys oder anderen Sprechfunksystemen, und ihren Basisstationen ausgeht, abzuschwächen bzw. abzuschirmen.

## Beschreibung

Die Erfindung betrifft Textilgewebe im allgemeinen, insbesondere aber Kleidungstücke jeglicher Art, welche Materialien enthalten, die elektromagnetische Felder (EMF) zu dämpfen vermögen, und ihre Verwendung zur Abschirmung von hochfrequenter Strahlung (HF-Strahlung). Derartige Gewebe sind somit hervorragend geeignet, Strahlung, welche von Radio- oder Mikrowellen emittierenden Geräten und Einrichtungen, insbesondere Mobiltelefone, Handys oder anderen Sprechfunksystemen, und ihren Basisstationen ausgeht, abzuschwächen bzw. abzuschirmen.

Radio- und Mikrowellen gehören zur hochfrequenten elektromagnetischen Strahlung (HF-Strahlung). Während Radiowellen eine Frequenz zwischen 0,5 MHz und etwa 150 MHz (z. B. UKW: 88 - 108 MHz) aufweisen, erstreckt sich der Frequenzbereich von Mikrowellen von etwa 300 MHz bis 300 GHz und umfaßt somit zahlreiche zur Zeit genutzte Anwendungsgebiete, insbesondere in der Nachrichtentechnik. Im unteren Bereich dieses Frequenzbandes (300 bis ca. 890 MHz werden Fernsehsendungen (UHF-Bereich) ausgestrahlt; Radargeräte arbeiten in einem Frequenzbereich von etwa 1 - 50 GHz, während Mikrowellengeräte für den Haushalt Strahlung in der Regel zwischen 2 - 4 GHz erzeugen. Neben dem sogenannten "C-Band" (400 - 600 MHz) benutzen moderne Mobilfunkgeräte zur Zeit vor allem Frequenzen in drei unterschiedlichen Bändern: 890 - 960 MHz, etwa 1750 - 1850 MHz und etwa 1890 - 2000 MHz. Eine neue Generation von mobilen drahtlosen Telefonen wird bei einer Frequenz von über 2000 MHz senden.
Sprechfunkeinrichtungen, wie CB-Funk ("Walkie-Talkies") oder Polzeifunk arbeiten in der Regel in Frequenzbereichen der Radiowellen, nämlich zwischen etwa 20 und 80 MHz.

Während früher schwere und unhandliche Geräte vorwiegend im militärischen und nicht-öffentlichen Bereichen (Polizei, Zoll, etc.) nur einem kleinen Personenkreis zugänglich war, fanden in den letzten Jahren immer kleinere, leichtere und preiswertere Mobiltelefone ("Handys") bei einem immer größer werdenden Publikum für den privaten und geschäftlichen Einsatz großen Anklang, zumal der Ausbau flächendeckender Netze - eine Grundvoraussetzung für die Kommerzialisierung dieser Technologie - kontinuierlich vorangetrieben wurde. Zur Zeit sind weltweit etwa 400 Millionen solcher Geräte im Einsatz, und ihre Zahl wird sich in drei bis vier Jahren etwa verdoppeln.

Somit wird auch die Mikrowellenstrahlung, insbesondere in dem Bereich zwischen 400 und 4500 MHz im alltäglichen Leben erheblich zunehmen. Mobiltelefone bzw. Sprechfunkeinrichtungen der genannten Art unterscheiden sich insofern von den meisten anderen HF erzeugenden Geräten des Alltags, dass sie am oder in unmittelbarer Nähe des Körpers getragen bzw. verwendet werden müssen und die Dauer der Strahleneinwirkung auf insbesondere Kopf- und Brustbereich zum Teil erheblich ist. Die Frage, ob durch die zunehmende HF-Strahlung, die Gesundheit gefährdet ist, wird in letzter Zeit verstärkt diskutiert.

HF - Felder werden im allgemeinen von der Antenne des Sendeteils ausgestrahlt. Die elektrischen und magnetischen Felder können in Abhängigkeit von der Frequenz mit den entsprechenden Feldern von elektrisch geladenen Teilchen im menschlichen Körper in Wechselwirkung treten. Elektrische Ladungen treten aber auch in bestimmten Molekülen / lonen sowie beispielsweise an Zellwänden auf. Nervenreize basieren auf Änderungen von elektrischen Potentialen an den Synapsen und das menschliche Gehirn erzeugt selbst messbare elektrische Felder. All solche Ladungen oder Felder können unter Umständen mit den äußeren, durch HF-Strahlung erzeugten Feldern in Resonanz treten und biologische Veränderungen bewirken.

Wie tief die HF - Strahlung in biologisches Gewebe eindringt, hängt von der Sendefrequenz ab. Bei den heute gängigen Mobilfrequenzen von ungefähr 890/960 bzw. 1800/1900 MHz beträgt die Eindringtiefe in den menschlichen Körper mehre Zentimeter (im C-Band darüber), bei den höher frequenten Systemen der neuen Generation (1900 - 2070 und 2100 - 2300, UMTS) liegt sie entsprechend geringer. Bei hochfrequenten Systemen (über 10 GHz), wie Radareinrichtungen, kann eine Eindringtiefe von lediglich 1 mm und darunter festgestellt werden. Zu beachten ist jedoch, dass erwiesenermaßen beim Telefonieren auch die der Antenne gegenüberliegende Kopfseite exponiert wird, und meßbare Strahlung aus dem Kopf wieder austritt.

Mikrowellenstrahlung im Funksendebereich (Fernsehen, Mobiltelefone) erzeugen in biologischem Gewebe vor allem eine mehr oder weniger ausgeprägte Wärmeentwicklung. Diese Wärmeentwicklung kann auch bei intensiver Einwirkung physisch wahrgenommen werden. Da die Wärmeentwicklung maximal nur zu einer lokalen Temperaturerhöhung von etwa 1°C führt, sollte ihr ein ernsthafter pathologischer Effekt jedoch abzusprechen sein.

Seit geraumer Zeit werden jedoch auch HF - Strahlungseffekte diskutiert, welche auf nicht-thermischen Ursachen beruhen sollen. Tatsächlich wird verbreitet von zum Teil unspezifischen gesundheitlichen Irritationen, wie Kopfschmerzen, Gedächtnisschwäche, Müdigkeit, motorische Störungen, Schwindel, Herzrhythmusstörungen und Unwohlsein berichtet, die mit der exzessiven Benutzung von Mobiltelefonen in Verbindung gebracht werden.

Teilweise widersprüchliche Untersuchungen an Tieren, welche einer permanenten erhöhten Mikrowellenstrahlung in einer gewöhnlichen Mobiltelefonen üblichen Intensität ausgesetzt wurden, berichten von einer Erhöhung von Enzymaktivitäten, Bluthochdruck, Zunahme der Gehirnstromtätigkeit, Veränderungen der Permeabilität der Blut-Gehirn Schranke, Veränderung der REM - Phase, der Hormonproduktion sowie Auslösung von Alzheimer-Symptomen und sogar Initiierung von Tumorwachstum. Obwohl die Energie von Mikrowellenstrahlung nicht ausreichen dürfte, um Atome zu ionisieren oder chemische Bindungen von Molekülen zu spalten, wurde Befunde publiziert, nach denen eine Zunahme an DNA - Kettenverkürzungen unter Einwirkung von HF - Strahlung bei Ratten festgestellt wurde.

Für all diese Untersuchungen gibt es zur Zeit noch keine oder keine allgemein anerkannte Rationale, insbesondere weil die durch HF-Strahlung vorrangig im Mikrowellenbereich biologisch aufgenommene Strahlenleistung nach gegenwärtigem Verständnis zu gering erscheint, um die genannten prognostizierten zum Teil erheblichen Wirkungen zu erklären. Die aufgenommene Strahlenleistung (Spezifische Absorptionsrate SAR, W / kg) kann aber trotz verhältnismäßiger geringer Ausgangsleistung des Senders vor allem bei längerer Benutzung oder längerem Tragen der mobilen Telekommunikationseinheit im eingeschalteten Zustand beträchtlich sein (Dauerbelastung). Die SAR berücksichtigt nämlich nicht nur den Betrag der übertragenen HF - Energie, sondern auch in welcher Zeit diese Energie auf eine bestimmte Masse biologischen Gewebes übertragen wird. Die SAR und ihre Verteilung im Körper ist von vielen Faktoren abhängig. Neben der Frequenz spielen räumliche und zeitliche Veränderungen des Feldes eine Rolle. Daneben sind elektrische Eigenschaften und Strukturen des biologischen Gewebes, welche eigene elektromagnetischen Felder in ihnen generieren, die mit dem externen Sendefeld in Resonanz treten können, von Bedeutung. So nimmt zum Beispiel Knochengewebe die Energie anders auf als gut durchblutetes Muskelgewebe. Auch können individuell unterschiedliche Physiologien zu unterschiedlichen Ladungszuständen und damit Wechselwirkungen und Veränderungen in bestimmten Körperbereichen und Zellen führen. Der Einfluß und das Zusammenwirken elektrischer bzw. elektromagnetischer lokaler Mini- und Mikrofelder auf biologische oder pathologische Veränderungen ist bislang nicht ausreichend untersucht worden. Erschwerend kommt hinzu, dass die SAR meßtechnisch nicht einfach zu erfassen ist. Aus diesem Grund wird in der Praxis oft mit abgeleiteten Feldgrößen, wie der Leistungsflußdichte (W/m²) oder der elektrischen, bzw. magnetischen Feldstärke ( V / m bzw. A / m) gearbeitet, wodurch zuweilen nicht vergleichbare Versuchsanordnungen und Ergebnisse resultieren. So entspricht einer SAR von 1 - 4 W/ kg, dies ist etwa der Durchschnittswert der Strahlungsaufnahme im Körper eines Erwachsenen bei Benutzung eines Mobiltelefons (950 MHz), ca. 20 bis 40 V / m.

Unabhängig von der teilweise kontroversen und wissenschaftlich nicht einheitlichen Lage erscheint es notwendig, bei der Benutzung von Mobiltelefonen oder anderen Sprechfunkeinheiten, welche im HF-Bereich in der Regel in unmittelbarer Körpernähe senden, Vorsichtsmaßnahmen zu ergreifen, um sowohl nachweislich auftretende jedoch oft unspezifische subjektive Symptome als auch eventuell durch HF - Strahlung ausgelöste Krankheiten zu vermeiden. Aufgabe dieser Erfindung war es somit, einen hinreichenden Schutz für Personen, welche betriebsbereite mobile Telefone in unmittelbarer Nähe zu ihrem Körper tragen oder benutzen, bereit zustellen. Hierbei ergibt sich das Problem, dass der naheliegende Schutz, die Geräte selbst durch die hierfür üblichen und bekannten Maßnahmen abzuschirmen, nicht oder ungenügend realisierbar ist, da hierdurch die Sende- und Empfangsleistung unterbunden oder stark beeinträchtigt wird. Damit kann der Schutz nur am oder beim Benutzer des mobilen Telefons vorgenommen werden.

Gegenstand der Erfindung ist somit die Verwendung von Geweben auf Basis von Textilfasern und mindestens einer Komponente, welche aus einem elektromagnetische Felder (EMF) dämpfenden Material besteht, zur Abschirmung von hochfrequenter (HF) Strahlung, die von drahtlosen Mobiltelefonen und Sprechfunkeinrichtungen emittiert wird. Erfindungsgemäß ist hierbei auch jene Strahlung mit eingeschlossen, welche von den Basisstationen oder Verstärkereinheiten ausgestrahlt wird, auch wenn diese in der Regel nur in unmittelbarer Nähe der Sendeeinrichtungen / Sendemasten eine Gefahr darstellen sollte.

HF-Strahlung im Sinne der Erfindung umfaßt elektromagnetische Strahlung im Radiowellenbereich zwischen 20 und 100 MHz, vorzugsweise zwischen 20 und 50 MHz, insbesondere zwischen 20 und 35 MHz, sowie Mikrowellenstrahlung zwischen 400 und 5000 MHz. Letztere umfaßt vorzugsweise die Frequenzbänder von 400 bis 650 MHz, 850 bis 1000 MHz, 1500 bis 2300 MHz und 3000 bis 4500 MHz. Hierunter sind wiederum die Bereiche 880 bis 960 MHz, 1750 bis 1890 MHz sowie 1900 bis 2300 MHz von besonderem Interesse.

Gegenstand der Erfindung ist somit insbesondere die besagte Verwendung zur Abschirmung von Mikrowellenstrahlung im Bereich von 400 bis 4500 MHz (Mobilfunk) und zur Abschirmung von Radiostrahlung in Bereich von 20 bis 50 MHz (Sprech- / CB- Funk).

Die erfindungsgemäßen Gewebe bestehen im wesentlichen aus herkömmlichen Textilfasern und mindestens einer weiteren Materialkomponente, welche elektromagnetische Felder, die von hochfrequenter Strahlung der genannten Frequenzen erzeugt wird, ganz oder teilweise abzuschirmen vermag. Dieses Gewebe weist je nach Zusammensetzung einen Dämpfungsfaktor von 2 bis 1000, vorzugsweise 5 bis 500 auf, insbesondere 5 bis 100. Zuweilen sind auch schon Dämpfungsfaktoren von 2 bis 15 ausreichend, was einen nur geringen Anteil (unter 3%) des EMF - abschirmenden Materials erforderlich macht.

Als Dämpfungsfakor im Sinne der Erfindung wird jener Faktor bezeichnet, um den die elektrische oder magnetische Feldstärke (und die damit indirekt in Verbindung stehenden Größen, siehe oben) durch die Materialeigenschaften reduzieren werden kann. Ein Dämpfungsfakor von beispielsweise 5 besagt, dass z. B. die Feldstärke eines bestimmten Ausgangswertes (ungedämpft) bei Durchtritt durch das erfindungsgemäße Material auf ein Fünftel herabgesetzt wird. Der Dämpfungsfaktor kann auch auf eine in Dezibel (dB) angegebene Leistungsdämpfung umgerechnet werden. Die Leistungsdämpfung ist hierbei das Zehnfache des dekadischen Logarithmus des Dämpfungsfaktors. Eine Leistungsdämpfung von beispielsweise 30db entspricht einen Dämpfungsfaktor von etwa 1000.

Gegenstand der Erfindung ist somit die Verwendung von entsprechenden Geweben, welche dadurch gekennzeichnet sind, dass sie ein EMF - dämpfendes Material aufweisen, welches einen Dämpfungsfakor des Gewebes zwischen 2 und 1000, insbesondere zwischen 5 und 100 bewirkt.

Als EMF-dämpfende Materialien eignen sich Metalle, Halbleitermaterialien oder entsprechende polymere Stoffe.

Als erfindungsgemäße Metalle sind im Prinzip alle gängigen bekannten Metalle zu nennen. Bevorzugt sind jedoch vor allem edle Metalle, also solche, die einen positiven Wert des Normalpotentials ε₀ (V) (in saurer Lösung) aufweisen; dies sind insbesondere Kupfer, Silber, Platin oder Gold. Von den weniger bevorzugten, aber prinzipiell geeigneten unedlen Metallen (negatives ε₀) sind Eisen und Nickel zu nennen. Erfindungsgemäß sind auch Legierungen der genannten edlen Metalle aber auch Legierungen von edlen Metallen mit unedlen Metallen bestens geeignet, so beispielsweise Ag/Cu-, Ag/Au, Au/Cu-, Cu/Ni-, Cu/Fe-, Ag/Ni-, Cu/Pt-, Fe/Pt-, Cu/Ag/Au-. Cu/Fe/Ni- Legierungen.

Als Halbleitermaterialien im Sinne der Erfindung sind solche Stoffe zu verstehen, die ihre Leitfähigkeit durch vorhandene Störstellen in ihrer Kristallstruktur erhalten. Dies sind beispielsweise Materialien, die Silicium, Germanium, Bor oder Selen enthalten. Auch nicht - leitfähige Materialien, welche durch Einbau von Dotierstoffen elektrische Leitfähigkeit erlangen, sind erfindungsgemäß mit umfaßt. Solche Materialien und ihre Herstellung sind hinlänglich bekannt und in der Literatur zahlreich beschrieben.

Als Polymermaterialien im Sinne der Erfindung gelten polymere Stoffe, die elektrische Leitfähigkeit oder Absorption elektromagnetischer Felder zeigen. In der Regel sind dies Polymere, die eine erhöhte Anzahl von hydrophilen Gruppen aufweisen. Beispiele für geeignete Stoffe sind Polyanilin, Polypyrrol, Polyacetylen, Poly(para)phenylen oder Poly(ortho)toluiden, die gegebenenfalls ebenfalls dotiert sein können. Über weitere Stoffe und ihre physikalischen, insbesondere elekromagnetischen Eigenschaften wird ausführlich bei "Microvave Properties of Conductive Polymers" (Handbook of Organic Conductive Molecules and Polymers, Vol. 3, 1997, John Wiley & Sons) berichtet.

Bevorzugter Gegenstand der Erfindung ist jedoch eine Verwendung von entsprechenden Geweben, welche als EMF - abschirmendes Material Metall enthalten, worin das Metall Kupfer, Silber, Gold, Platin oder eine Legierung dieser Metalle untereinander oder eine Legierung dieser Metalle mit anderen Metallen, vorzugsweise Nickel und / oder Eisen ist.

Die EMF - dämpfenden Materialien können auf unterschiedliche Weise in das Gewebe ein- oder aufgebracht werden.
Eine Möglichkeit besteht darin, das Material in Form von Fasern bereitzustellen, die beim Herstellen des Gewebes mit den herkömmlichen Gewebefasern verwoben werden. Die Fasern selbst können hierbei vollständig aus dem betreffenden Material sein, also Metallfasern, Halbleitermaterial-Fasern oder Polymerfasern. Das Material kann aber auch in Form von Partikeln, beispielsweise Blättchen, Kügelchen oder Gitternetzstrukturen vorliegen. Diese Partikel können dann direkt auf oder zwischen die herkömmlichen Gewebefasern eingebracht werden. Die Partikel können aber auch zunächst in geeigneten Trägermaterialien, beispielsweise herkömmliche, vorzugsweise für Textilien geeignete Polymere bei deren Herstellung aufgenommen werden. Diese mit den besagten Partikeln beladenen Trägerpolymere können entweder zu eigenen Fasern gezogen werden, die mit den herkömmlichen Gewebefasern verarbeitet werden, sie können aber auch als Beschichtungsmaterial für die herkömmlichen Gewebefasern dienen. Letztlich können die herkömmlichen Gewebefasern aber auch unmittelbar, d.h. ohne Verwendung eines zusätzlichen Trägermaterials beschichtet werden. Die jeweilige Beschichtung (z.B. durch Bedampfen oder Besprühen) erfolgt dabei nach gängigen in der Textilindustrie bekannten Techniken, wobei die Beschichtung der einzelnen Fasern wie auch des fertigen Gewebes erfolgen kann.
Vorzugsweise liegt das EMF - dämpfende Material in Form von Fasern vor, welche mit den herkömmlichen Gewebefasern bei der Herstellung des Gewebes miteinander verwoben werden.

Liegt das EMF - dämpfende Material in Form von Fasern vor oder in Partikeln, welche in zu Fasern gezogenen Trägermaterialien eingebettet sind, sollte der Durchmesser dieser Fasern erfindungsgemäß 10 bis 100 µm, vorzugsweise 10 bis 50 µm, insbesondere 15 bis 35 µm betragen. Diese Durchmesser gewährleisten nicht nur eine ausreichende Reflektion des einstrahlenden elektromagnetischen Feldes, sondern auch eine gute Bearbeitung und komfortable Eigenschaften des Gewebes.

Der Anteil des EMF - abschirmenden Materials am Gesamtgewebe hängt von dem gewünschten zu erzielenden Dämpfungsfaktor ab. Dieser wiederum ist abhängig von der Energie (Frequenz) der emittierten Strahlung. Überraschenderweise wurde gefunden, dass ein Dämpfungsfakor von 20 (10dB) bis 1000 (30dB) bei einer Strahlung zwischen 400 und 2300 MHz bereits bei einem Anteil des EMF - abschirmende Material von 5 bis 15 %, vorzugsweise 8 bis 12%, erreicht werden kann (15% bedeuten hier: 15 von 100 Fasern bestehen aus oder enthalten das EMF - abschirmende Material). Ein Dämpfungsfaktor zwischen 2 und 15, vorzugsweise zwischen 4 und 10, kann bereits mit einem entsprechenden Anteil von 1 bis 5% erzielt werden. Die Anteile beziehen sich hier auf Anteile Fasern des EMF - abschirmenden Materials zu den Gesamtfasern des Gewebes. Auch bei Einsatz von Partikeln in welcher Form auch immer, sind bei den genannten Gehalten (1 - 15% bezogen auf das Gewebe, hier w/w) vergleichbare Dämpfungsfaktoren zu erzielen. Strahlung mit höheren Frequenzen (Energie) wird durch die erfindungsgemäßen Materialien stärker gedämpft als durch Strahlung beispielsweise im Radio- oder niederen Mikrowellenbereich.

Als herkömmliches Gewebe im Sinne der Erfindung wird Gewebe verstanden, dass alle Arten von Textilien im weitesten Sinne mit einschließt. Vorzugsweise eignen sich alle Naturfasern, insbesondere Wolle, Baumwolle, Leinen oder Seide, bzw. Gemische aus diesen Fasern. Die Natur fasern lassen sich problemlos mit den EMF - abschirmenden Fasern verarbeiten, bzw. letztere lassen sich ohne weiteres in diese einbringen. Mit einem Anteil von bis zu 15%, insbesondere zwischen 1 und 5% bewahren die Gewebe sowohl optisch als auch haptisch ihre ursprüngliche Qualität und sind somit generell einsetzbar. Das Gewebe kann aber auch, falls besondere Anfordernisse an Wasserabweisung oder Knitterfestigkeit gestellt werden, ganz oder teilweise auf Basis herkömmlicher textiler Polymerfasern, wie z.B. Polyester, Polyacryl, Polyethylen etc., aufgebaut sein.

Die textilen Gewebe können erfindungsgemäß zu Kleidungsstücken, z. B. in Form von Hemden, Blusen, Jacken, Mänteln, Hals- und Kopftüchern und Kopfbedeckungen jeglicher Art verarbeitet sein. Es besteht aber auch die Möglichkeit Einlagen aus dem erfindungsgemäßen Gewebe einzusetzen, beispielsweise in Taschen, z. B. Brust- und Jackentaschen, in denen sehr oft das betriebsbereite Handy aufbewahrt bzw. getragen wird. Hier wird die Einlage zweckmäßigerweise zwischen dem Mobiltelefon und dem Körper angebracht. Auch der Schutz beim Telefonieren, also im Kopf-, bzw. Ohrbereich kann durch Verwendung von geeigneten Kopfbedeckungen oder Stirnbändern hergestellt werden.

### Beispiel 1:

Die Dämpfung der elektromagnetischen Feldstärke durch ein erfindungsgemäßes Gewebe wird gemessen. Hierbei wird ein elekromagnetisches Wechselfeld einer bestimmten Frequenz, die einer Frequenz eines Mobiltelefons oder einer Sprechfunkeinrichtung entspricht, mittels eines HF-Signalgenerators und eines HF-Verstärkers erzeugt. Zwischen dem Generator und einer mit einem Feldstärkemeßgerät versehenen Sonde ist das jeweilige EMF - abschirmende Gewebe angeordnet, und zwar so, dass das Gewebe unmittelbar an oder über der Sonde angebracht ist. Als Ausgangswert wird eine Feldstärke von 10 V/m generiert. Dies entspricht etwa der Feldstärke, die in unmittelbarer Nähe (3 - 5 cm) der Antenne eines üblichen Mobiltelefons (400 - 2000 Mhz) auftritt. Die Dämpfung wird in dB angegeben. Zur Umrechnung in Dämpfungsfaktoren siehe oben.

### Beispiel 2:

Die in Beispiel 1 beschriebene Anordnung wird benutzt, um die Dämpfung bei 900 MHz Erregerfrequenz für ein Baumwollgewebe, das eine Metallfaser zu 4% (96 Baumwollfasern, 4 Metallfasern) enthält. Die Metallfaser (30 µm) ist eine Kupfer / Silber - Legierung (75/25 w/w).
Gewebe in unmittelbarer Nähe (1 - 3 cm) der Meßsonde:
   Dämpfung bei horizontaler Ausrichtung der Antenne: 15 (dB)
   Dämpfung bei vertikaler Ausrichtung der Antenne: 5 (dB).
Gewebe in 10 - 15 cm der Meßsonde:
   Dämpfung bei horizontaler Ausrichtung der Antenne: 8 (dB)
   Dämpfung bei vertikaler Ausrichtung der Antenne: 1 (dB).

### Beispiel 3:

Wie Beispiel 2, jedoch 1800 MHz Erregerfrequenz.
Gewebe in unmittelbarer Nähe (1 - 3 cm) der Meßsonde:
   Dämpfung bei horizontaler Ausrichtung der Antenne: 20 (dB)
   Dämpfung bei vertikaler Ausrichtung der Antenne: 26 (dB).
Gewebe in 10 - 15 cm der Meßsonde:
   Dämpfung bei horizontaler Ausrichtung der Antenne: 9,5 (dB).

### Beispiel 4:

Die in Beispiel 1 beschriebene Anordnung wird benutzt, um die Dämpfung bei 900 MHz Erregerfrequenz für ein Baumwoll-/Leinengewebe (75/25), das eine Polymerfaser (65 µm), welche Kupferblättchen (ca. 20 µm) enthält (Polmer / Kupfer 65 / 35 w/w), aufweist. Der Anteil der Polymerfaser beträgt 20% (80 Naturfasern und 20 Polymer-Kupfer - Fasern).
Man erhält Dämpfungsfaktoren zwischen 8 und 20.

## Patentansprüche

1. Verwendung von Geweben auf Basis von Textilfasern und mindestens einer Komponente, welche aus einem elektromagnetische Felder (EMF) dämpfenden Material besteht, zur Abschirmung von hochfrequenter (HF) Strahlung, die von drahtlosen Mobiltelefonen und Sprechfunkeinrichtungen einschließlich ihrer Basisstationen emittiert wird.

2. Verwendung nach Anspruch 1 zur Abschirmung von Mikrowellenstrahlung im Bereich von 400 bis 4500 MHz (Mobilfunk).

3. Verwendung nach Anspruch 1 zur Abschirmung von Radiostrahlung in Bereich von 20 bis 50 MHz (Sprech- / CB- Funk).

4. Verwendung nach einem der Ansprüche 1 bis 3, dadurch gekennzeichnet, dass das EMF - dämpfende Material einen Dämpfungsfakor zwischen 2 und 1000 bewirkt.

5. Verwendung nach Anspruch 4, dadurch gekennzeichnet, dass das EMF-dämpfende Material einen Dämpfungsfakor zwischen 5 und 100 bewirkt.

6. Verwendung nach einem der Anspruch 4, dadurch gekennzeichnet, dass das EMF - dämpfende Material ausgewählt ist aus mindestens einem Metall und / oder einem Halbleitermaterial und / oder einem Polymerstoff.

7. Verwendung nach Anspruch 6, dadurch gekennzeichnet, dass das EMF - dämpfende Material ein Metall ist.

8. Verwendung nach Anspruch 7, dadurch gekennzeichnet, dass das Metall Kupfer, Silber, Gold, Platin oder eine Legierung dieser Metalle untereinander oder eine Legierung dieser Metalle mit anderen Metallen ist.

9. Verwendung nach Anspruch 4, dadurch gekennzeichnet, dass das EMF - dämpfende Material in Form von Fasern, welche mit den Textilfasern verwoben sind, oder in Form von gesonderten Partikeln oder Strukturen, welche in das Textilgewebe eingebracht sind, vorliegt, oder dass die Textilfasern mit besagtem EMF - dämpfende Material beschichtet sind.

10. Verwendung nach Anspruch 9, dadurch gekennzeichnet, dass das EMF - dämpfende Material in Form von Fasern vorliegt.

11. Verwendung nach Anspruch 10, dadurch gekennzeichnet, dass die Fasern einen Durchmesser von 10 - 100 µm aufweisen.

12. Verwendung nach einem der vorangehenden Ansprüche, dadurch gekennzeichnet, dass als Textilfasern Baumwolle, Wolle, Leinen, Seide oder Mischgewebe dieser Fasern eingesetzt werden, wobei Anteile von Kunstfasern enthalten sein können.
